Europäisches Patentamt

European Patent Office

Office européen des brevets

(11) Publication number: **0 160 852 B1**

## EUROPEAN PATENT SPECIFICATION

(45) Date of publication of patent specification: **11.03.92**    (51) Int. Cl.⁵: **C12Q 1/04**

(21) Application number: **85104306.7**

(22) Date of filing: **10.04.85**

(54) **Medium for arginine decarboxylation test.**

(30) Priority: **09.05.84 JP 91063/84**

(43) Date of publication of application:
**13.11.85 Bulletin 85/46**

(45) Publication of the grant of the patent:
**11.03.92 Bulletin 92/11**

(84) Designated Contracting States:
**BE DE FR GB IT**

(56) References cited:
**US-A- 4 335 205**

**CHEMICAL ABSTRACTS, vol. 98, 1983, page
323, abstract no. 68494t, Columbus, Ohio,
US; & JP-A-57 146 596 (NIPPON TECHTRON
K.K.) 10-09-1982**

(73) Proprietor: **TERUMO KABUSHIKI KAISHA trad-
ing as TERUMO CORPORATION
44-1, 2-chome, Hatagaya Shibuya-Ku
Tokyo 151(JP)**

(72) Inventor: **Igarashi, Takeshi
31-13, 1-chome, Suwa
Tama-shi Tokyo(JP)**
Inventor: **Endo, Toshihiko
1417-1, Miyajima
Fuji-shi Shizuoka-ken(JP)**
Inventor: **Koshi, Isei
3350-1, Mannohara-Shinden
Fujinomiya-shi Shizuoka-ken(JP)**

(74) Representative: **Henkel, Feiler, Hänzel & Part-
ner
Möhlstrasse 37
W-8000 München 80(DE)**

**Description**

BACKGROUND OF THE INVENTION

Field of the Invention:

This invention relates to a mixture for testing a microorganism for biochemical behavior. More particularly, this invention relates to an improved mixture to be used in performing arginine decarboxylation test on microorganisms such as enteric bacteria by the method of biochemical identification.

For administration of a proper medication to a patient of an infectious disease, it is essential to identify a pathogenic microorganism responsible for the disease, subjecting the patient to a sensitivity test, and selecting an effective medicine. In the identification of such a pathogenic microorganism, many biochemical tests are performed. One of them is an arginine decarboxylation test. The mixture of this invention is advantageously used for the arginine decarboxylation test.

Description of the Prior Art:

The arginine decarboxylation test is intended to detect a microorganism which decarboxylates L-arginine into agmatine and carbon dioxide. At present, it is one of the most important tests performed on microorganisms for biochemical behavior. This test is carried out by culturing a given microorganism on a medium containing L-arginine and a pH indicator as main components. As the medium to be used in this test, a composition formed of casitone, glucose, monosodium dihydrogen phosphate, L-arginine hydrochloride, bromothymol blue, and purified water has been recently proposed [Japanese Patent Publication No. SHO 58(1983)-20,263]. This medium permits the time required for culture to be notably decreased as compared with the conventional countertype. Specifically, the culture time has been one to two days in the conventional medium, whereas it is four to five hours in the medium under discussion, making it possible to conduct the test and find the result of the test on one same day. Depending on the kind of the microorganism under test, however, the culture performed for four to five hours in this medium does not permit the reaction to proceed sufficiently for required evaluation. In this case, the culture time must be elongated and the evaluation of the test result made on the following day. In the circumstance, the desirability of developing a medium in which any microorganism can be cultured sufficiently in a matter of four to five hours to permit clear distinction between positive and negative test of the reaction has found growing approval.

For early medication, the identification of a pathogenic microorganism is desired to be carried out as rapidly as permissible. There are, however, times when the evaluation of the test result is compelted to be performed on the following day because of the convenience of the work involved in the test. In this case, the medium is desired to be such that the culture time is not rigidly specified and, therefore, the culture time may be elongated to suit the convenience of the work and, despite the elongation of the culture time, the reaction is not suffered to proceed excessively and the evaluation of the test result can be conducted accurately.

For the efficiency of work involved, many bio-chemical tests are frequently performed all at once by the use of a multi-well culture plate. Thus, it becomes necessary for culture times of different test items to be equalized to one another. Again in this case, the culture medium is desired not to involve any rigid limitation of culture time.

An object of this invention, therefore, is to provide a novel medium for arginine decarboxylation test.

Another object of this invention is to provide a medium which does not specifically limit culture time and enables the identification of the pathogenic microorganism to be performed on the same day as the culture is carried out or on the day following the culture.

A further object of this invention is to provide a medium for arginine decarboxylation test which permits a color reaction to produce a distinct color and enables the evaluation of test result to be effected with ease.

SUMMARY OF THE INVENTION

The objects described above are attained by a mixture for the arginine decarboxylation test which is composed of 1 to 10 g of yeast extract, 1 to 10 g of casein-trypsin hydrolyzing peptone, 0.1 to 2 g of glucose, 0.2 to 2 g of a monoalkali metal dihydrogen phosphate, 5 to 50 g of L-arginine mineral acid salt, 0.005 to 0.1 g of 5'-pyridoxalphosphoric ester and 0.01 to 0.2 g of a pH indicator.

When dissolved in 1 liter of water, said mixture provides a culture medium assuming a pH-value in the

2

range of 5.5 to 6.5, preferably 5.8 to 6.2.

DESCRIPTION OF THE PREFERRED EMBODIMENT

The medium prepared by using the mixture of the present invention is an aqueous solution of yeast extract, casein-trypsin hydrolyzing peptone, glucose, monoalkali metal dihydrogen phosphate, L-arginine mineral acid salt, 5'-pyridoxalphosphoric ester and a pH indicator.

The mixture of the present invention comprises preferably 3 to 7 g of yeast extract, preferably 3 to 7 g of casein-trypsin hydrolyzing peptone, preferably 0.2 to 1.0 g of glucose, preferably 0.3 to 1.0 g of monoalkali metal dihydrogen phosphate, preferably 10 to 40 g of L-arginine mineral acid salt, preferably 0.001 to 0.08 g of 5'-pyridoxalphosphoric ester and preferably 0.04 to 0.1 g, of a pH indicator.

5'-Pyridoxalphosphoric ester possesses an ability to promote the decarboxylation of arginine and serves to shorten the culture time. The yeast extract is intended to promote the enzymatic reaction.

The monoalkali metal dihydrogen phosphate is a pH adjuster. The alkali metal moiety of this phosphate is desired to be potassium or sodium. The L-arginine mineral acid salt is a substrate. The mineral acid salt is desired to be hydrochloride, sulfate, nitrate or phosphate. The pH indicator is not specifically limited. Examples of the pH indicator suitable for this invention include phenol red, bromothymol blue, bromocresol purple, cresol red bromophenol red, chlorophenol red and the like. Among the pH indicators enumerated above, phenol red proves particularly desirable in the sense that the color produced in the color reaction is distinct enough to permit easy discrimination between positive and negative test even when the sample size is small.

The proportions of the components in the mixture of the present invention are critical. In particular, the component of the monoalkali metal dihydrogen phosphate is fixed so that the pH value of the medium, prepared by dissolving the mixture of the present invention in 1 l water will fall in the range of 5.5 to 6.5, preferably 5.8 to 6.2, and the color of the color reaction will appear distinctly. Also the proportions of the arginine and the 5'-pyridoxalphosphoric ester are selected so as to permit reduction in the culture time.

The proportion of the pH indicator may be varied more or less, depending on the particular kind of pH indicator to be selected. In the case of phenol red, for example, the proportion is desired to fall in the range of 0.04 to 0.1 g/l.

The mixture of this invention is prepared for use by dissolving the components in their respective proportions in water to provide a culture medium. In recent years, it is normal for numerous tests to be performed all at once by the use of a multi-well culture plate. For the medium prepared by using the mixture of this invention to be used in this manner, it is poured into the wells of the culture plate and dried to be used as dry medium. In the test, a suspension of a microorganism is dispensed in a prescribed volume to the individual wells and left standing therein to effect required culture for a prescribed length of time. By the change in color of the culture solution, the discrimination between positive test and negative test of the arginine decarboxylation reaction is effected.

The medium prepared by using the mixture of the present invention is used in much the same way as the conventional medium for arginine decarboxylation test. With said medium, although the culture time can be reduced to the order of three to five hours, it is not specifically limited to that range. The culture may be performed for a longer period. Thus, the medium permits the evaluation of the test result either on the same day as the sample is taken or on the following day. The color produced in color test is so distinct as to permit easy discrimination between positive test and negative test of the reaction.

Now, one working example of the actual use of the medium of this invention in the arginine decarboxylation test will be described below.

Examples I-VI and Controls I-III

| composition of mixture | Example | | | | | | Control | | |
|---|---|---|---|---|---|---|---|---|---|
| | I | II | III | IV | V | VI | I | II | III |
| Trypsin hydrolyzing peptone* | 5.0 | - | - | - | - | - | 10.0 | - | - |
| Yeast extract (g)** | 5.0 | 3.0 | 4.0 | 5.0 | 7.0 | 5.0 | - | 5.0 | 5.0 |
| Casein-trypsin hydrolyzing peptone (g)*** | 5.0 | 3.0 | 4.0 | 5.0 | 7.0 | 5.0 | - | 5.0 | 5.0 |
| Glucose (g) | 0.5 | 0.3 | 0.3 | 0.5 | 0.9 | 0.3 | 0.5 | 0.5 | 0.5 |
| Monosodium dihydrogen phosphate (g) | 0.7 | 0.4 | 0.5 | 0.7 | 1.0 | 0.3 | 0.8 | 0.7 | 0.2 |
| L-argininemonohydrochloride (g) | 30.0 | 15.0 | 20.0 | 30.0 | 40.0 | 30.0 | 30.0 | 30.0 | 30.0 |
| 5'-Pyridoxal phosphoric ester (g) | 0.05 | 0.03 | 0.02 | 0.05 | 0.08 | 0.05 | - | 0 | 0.2 |
| Bromothymol blue (g) | - | - | - | - | 0.1 | - | 0.12 | - | - |
| Phenol red (g) | 0.06 | 0.04 | 0.05 | 0.04 | - | 0.06 | - | 0.06 | 0.06 |
| Purified water (liter) for preparing the medium | 1 | 1 | 1 | 1 | 1 | 1 | 1 | 1 | 1 |
| pH of the medium | 6.0 | 6.0 | 6.0 | 6.1 | 6.2 | 5.9 | 6.0 | 6.0 | 6.0 |

* Trypsin hydrolyzing peptone produced by Difco.

** Yeast extract produced by Difco.

*** Casein-trypsin hydrolyzing peptone produced by Difco.

Culture conditions

The medium provided by using the mixture of this invention and cotrol media having the compositions as shown above were dispensed in a unit volume of 50 μl to the wells of a culture plate and dried at 40 °C.

4

Separately various microorganisms were cultured on agar culture plates at 35 ° to 37 °C for 18 to 24 hours and suspended in 1.0 ml sterilized distilled water in a concentration of about 6 to 9 x $10^8$ cells/ml. The suspensions were inoculated in a unit volume of 50 $\mu$l to the dry medium and, under a cover, cultured at 35° to 37 °C for a prescribed length of time. By the change in color of the resultant culture solutions, presence or absence of the arginine decarboxylation reaction was evaluated. The results are shown in Table 1.

Table 1

| Micro-organism | Medium of Example I | | Medium of Examples II-VI | | Control medium I | | Control medium II | | Control medium III | |
|---|---|---|---|---|---|---|---|---|---|---|
| | 4 to 5 hrs' culture | 16 to 20 hrs' culture | 4 to 5 hrs' culture | 16 to 20 hrs' culture | 4 to 5 hrs' culture | 16 to 20 hrs' culture | 4 to 5 hrs' culture | 16 to 20 hrs' culture | 4 to 5 hrs' culture | 16 to 20 hrs' culture |
| Aeromonas hydrophila | + | + | + | + | + | + | + | + | + | + |
| Enterobacter cloacae | + | + | + | + | − | + | + | + | + | + |
| Enterobacter cloacae | + | + | + | + | − | + | ± | + | ± | + |
| Enterobacter cloacae | + | + | + | + | − | + | ± | + | ± | + |
| Escherichia coli | − | − | − | − | − | − | − | − | − | − |
| Citrobacter freundii | − | − | − | − | − | − | − | − | − | − |
| Enterobacter aerogenes | − | − | − | − | − | − | − | − | − | − |
| Klebsiella pneumoniae | − | − | − | − | − | − | − | − | − | − |
| Serratia marcescens | − | − | − | − | − | − | − | − | − | − |

+:   Color of positive test
−:   Color of negative test
±:   Intermediate color closer to color of positive test

It is noted from the results of Table 1 that the use of the mixture of this invention in the arginine decarboxylation test permits accurate identification of microorganisms without reference to length of culture time. Thus, with the mixture of this invention, the evaluation of test result can be carried out either on the same day as the sample is taken or on the following day, depending on the convenience of the work involved in the test.

In contrast, in the use of control media on Enterobacter cloacae, for example, the color produced after 4 to 5 hours' culture is not amply distinct to permit the evaluation of test result on the same day as the sample is take. Moreover, the fact that the medium provided by using the mixture of this invention is not rigidly restricted by the length of culture time proves highly convenient where numerous biochemical tests are carried out all at once.

## Claims

1. A mixture for arginine decarboxylation test, composed of:
   1 to 10 g of yeast extract,
   1 to 10 g of casein-trypsin hydrolyzing peptone,
   0.1 to 2 g of clucose,
   0.2 to 2 g of monoalkali metal dihydrogen phosphate,
   5 to 50 g of L-arginine mineral acid salt,
   0.005 to 0.1 g of 5'-pyridoxalphosphoric ester and
   0.01 to 0.2 g of a pH indicator.

2. A mixture according to Claim 1, being composed of:
   3 to 7 g of yeast extract,
   3 to 7 g of casein-trypsin hydrolyzing peptone,
   0.2 to 1 g glucose,
   0.3 to 1 g of monoalkali metal dihydrogen phosphate,
   10 to 40 g of L-arginine mineral acid salt,
   0.01 to 0.08 g of 5'-pyridoxalphosphoric ester, and
   0.04 to 0.1 g of a pH indicator.

3. A mixture according to Claim 1, wherein said monoalkali metal dihydrogen phosphate is potassium salt or sodium salt.

4. A mixture according to Claim 1, wherein said L-arginine mineral acid salt is at least one salt selected from the group consisting of hydrochloride, sulfate and phosphate.

5. A mixture according to Claim 1, wherein said pH indicator is one compound selected from the group consisting of phenol red, bromothymol blue, bromocresol purple, cresol red, chlorophenol red and bromophenol red.

6. A mixture according to Claim 5, wherein said pH indicator is phenol red.

7. A culture medium exhibiting a pH value in the range of 5.5 to 6.5, preferably of 5.8 to 6.2, and for arginine decarboxylation test, obtained by dissolving a mixture of any of claims 1 to 6 in 1 liter of water.

## Revendications

1. Mélange destiné au test de décarboxylation de l'arginine, composé de :
   1 à 10 g d'extrait de levure,
   1 à 10 g de peptone hydrolysant la caséine-trypsine,
   0,1 à 2 g de glucose,
   0,2 à 2 g de dihydrogénophosphate de monométal alcalin,
   5 à 50 g de sel d'acide minéral de L-arginine,
   0,005 à 0,1 g d'ester 5'-pyridoxalphosphorique et
   0,01 à 0,2 g d'un indicateur de pH.

2. Mélange selon la revendication 1, composé de :
   3 à 7 g d'extrait de levure,
   3 à 7 g de peptone hydrolysant la caséine-trypsine,
   0,2 à 1 g de glucose,
   0,3 à 1 g de dihydrogénophosphate de monométal alcalin,
   10 à 40 g de sel d'acide minéral de L-arginine,

0,01 à 0,08 g d'ester 5'-pyridoxalphosphorique et
0,04 à 0,1 g d'un indicateur de pH.

**3.** Mélange selon la revendication 1, dans lequel ledit dihydrogénophosphate de monométal alcalin est un sel de potassium ou un sel de sodium.

**4.** Mélange selon la revendication 1, dans lequel ledit sel d'acide minéral de L-arginine est au moins un sel sélectionné dans le groupe constitué par le chlorhydrate, le sulfate de le phosphate.

**5.** Mélange selon la revendication 1, dans lequel ledit indicateur de pH est un composé sélectionné dans le groupe constitué par le rouge de phénol, le bleu de bromothymol, le pourpre de bromocrésol, le rouge de crésol, le rouge de chlorophénol et le rouge de bromophénol.

**6.** Mélange selon la revendication 5, dans lequel ledit indicateur de pH est du rouge de phénol.

**7.** Milieu de culture présentant un pH compris dans la plage de 5,5 à 6,5, de préférence de 5,8 à 6,2, et destiné au test de décarboxylation de l'arginine, obtenu par dissolution d'un mélange selon l'une quelconque des revendications 1 à 6 dans 1 l d'eau.

**Patentansprüche**

**1.** Mischung für einen Arginindecarboxylierungstest, bestehend aus
1 - 10 g Hefeextrakt,
1 - 10 g Casein-Trypsin-Hydrolysierpepton,
0,1 - 2 g Glucose,
0,2 - 2 g Monoalkalimetalldihydrogenphosphat,
5 - 50 g L-Argininmineralsäuresalz,
0,005 - 0,1 g 5'-Pyridoxalphosphorsäureester und
0,01 - 0,2 g eines pH-Indikators.

**2.** Mischung nach Anspruch 1, bestehend aus
3 - 7 g Hefeextrakt,
3 - 7 g Casein-Trypsin-Hydrolysierpepton,
0,2 - 1 g Glucose,
0,3 - 1 g Monoalkalimetalldihydrogenphosphat,
10 - 40 g L-Argininmineralsäuresalz,
0,01 - 0,08 g 5'-Pyridoxalphosphorsäureester und
0,04 - 0,1 g eines pH-Indikators.

**3.** Mischung nach Anspruch 1, dadurch gekennzeichnet, daß das Monoalkalimetalldihydrogenphosphat aus dem Kalium- oder Natriumsalz besteht.

**4.** Mischung nach Anspruch 1, dadurch gekennzeichnet, daß das L-Argininmineralsäuresalz zumindest aus einem Salz aus der Gruppe Hydrochlorid, Sulfat und Phosphat besteht.

**5.** Mischung nach Anspruch 1, dadurch gekennzeichnet, daß der pH-Indikator aus mindestens einer Verbindung aus der Gruppe Phenolrot, Bromthymolblau, Bromcresolpurpur, Cresolrot, Chlorphenolrot und Bromphenolrot besteht.

**6.** Mischung nach Anspruch 5, dadurch gekennzeichnet, daß der pH-Indikator aus Phenolrot besteht.

**7.** Kulturmedium eines pH-Werts im Bereich von 5,5 bis 6,5, vorzugsweise 5,8 bis 6,2, zur Durchführung eines Arginindecarboxylierungstests, erhalten durch Auflösen einer Mischung gemäß einem der Ansprüche 1 bis 6 in 1 l Wasser.